# EUROPEAN PATENT SPECIFICATION

(11) **EP 4 003 245 B1**
(45) Date of publication and mention of the grant of the patent: **20.08.2025**
(21) Application number: 20757956.6
(22) Date of filing: 24.07.2020
(51) Int. Cl.: A61F 5/01, A61F 5/058, A61F 5/34

(54) **IMPROVED MEDICAL APPARATUS FOR CONTAINING HAEMATOMAS**
VERBESSERTES MEDIZINISCHES GERÄT ZUR EINDÄMMUNG VON HÄMATOMEN
APPAREIL MÉDICAL AMÉLIORÉ POUR CONTENIR LES HÉMATOMES

(30) Priority: 25.07.2019 IT 201900012843
(43) Date of publication of application: 01.06.2022
(73) Proprietor: Jackstat Med S.r.l., Milan (IT)
(72) Inventor: Angeloro, Ciro, 26866 Sant'Angelo Lodigiano (Lodi) (IT); Morsillo, Enrico, 20078 San Colombano al Lambro (MI) (IT)
(74) Representative: Lunati & Mazzoni S.r.L.
(86) International application number: PCT/IB2020/056998
(87) International publication number: WO 2021/014414

(56) References cited:
- WO-A1-2016/001802
- WO-A2-2007/078845
- US-A1- 2012 330 091
- US-A1- 2013 110 019

## Description

The present invention relates to a medical apparatus for improved hematoma containment.

Performing a pacemaker or defibrillator implantation in a patient exhibits post-operative risks including the formation of a hematoma in the shoulder, especially in patients who undergo therapies with the use of anticoagulants or antiplatelet agents.

The formation of a hematoma close to the pocket of the pacemaker or defibrillator can lead to the development of an infection with possible damage to the cardiac tissue.

Another complication is the displacement of the implanted leads as a result of early or accidental patient mobilization.

To combat the aforementioned complications, the patient's shoulder is currently subjected to compression bandage, which must be carried out by skilled personnel in order to be performed correctly.

A medical apparatus for the containment of hematomas comprising a support unit enveloping at least a part of the patient's body and an inflatable cushion held in place by the support unit is currently known from patent application WO 2016/001802.

US 2012/330091 describes an orthopaedic device for treating bone fracture.

WO 2007/078845 describes orthopaedic devices for treating limb injury by reducing or milking edema.

However, the position of the cushion directly in contact with the patient's body is not comfortable for the patient and does not allow a uniform distribution of pressure over the whole part where the hematoma is present.

In view of the background art, it is the object of the present invention to provide a medical apparatus for the containment of hematomas which overcomes the aforementioned drawbacks.

According to the present invention, said object is achieved by a medical apparatus for the containment of hematomas for treatment following operations for implanting cardiac devices, said apparatus comprising a support unit externally enveloping at least a part of the patient's body and adapted to resist efforts aimed to detach said unit from said patient, at least one inflatable cushion held in place over or close to the patient's shoulder by said support unit, characterized in that it comprises a material arranged between said inflatable cushion and the patient's shoulder, said material being configured to distribute the pressure of said cushion, once inflated, on the patient's shoulder, said support unit comprising at least one pocket arranged to contain said material, said inflatable cushion being shaped so that, once inflated, it takes a wavy shape.

The features and advantages of the present invention will become apparent from the following detailed description of a practical embodiment thereof, shown by way of non-limiting example in the accompanying drawings, in which:
figure 1 is a front view of the medical apparatus for the containment of hematomas according to a first embodiment of the present invention;
figure 2 is a view of the internal part of the medical apparatus in figure 1;
figures 3 and 4 are views of the medical apparatus for the containment of hematomas according to a second embodiment of the invention worn by a patient;
figure 5 is a perspective view of the inflatable cushion of the apparatus in figures 3 and 4;
figure 6 is a section view of a part of the apparatus in figures 3 and 4 applied to a patient with the cushion inflated;
figure 7 is a top view of a part of the apparatus in figures 3 and 4 applied to a patient with the cushion inflated.

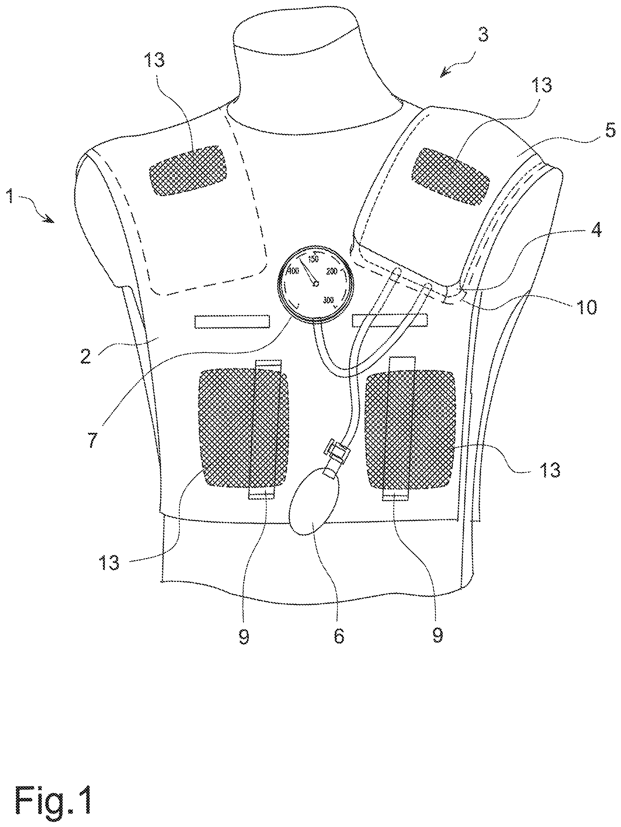

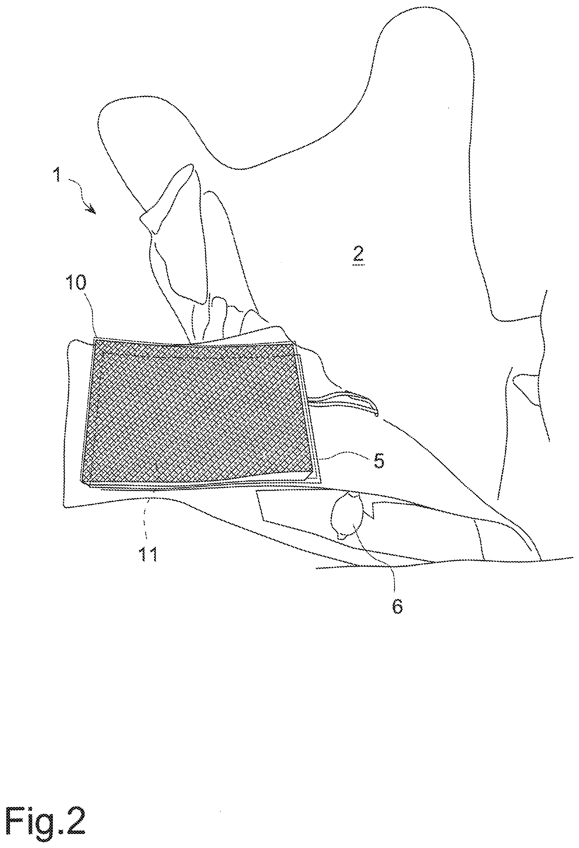

With reference to figures 1 and 2, a medical apparatus 1 for the containment of hematomas according to a first embodiment of the present invention is described.

The apparatus comprises a support unit 2 adapted to cover and externally envelop the patient's shoulder; preferably the support unit 2 is in the form of a jacket. The support unit 2 is provided in particular for treatments following operations for implanting a pacemaker or a defibrillator, i.e., when hematomas are possible at the shoulder.

The apparatus comprises at least one bag or cushion 4 preferably inflatable with air, substantially flattened and arranged at or straddling the shoulder; the inflatable bag or cushion is held in place on the shoulder by the support unit 2.

The apparatus comprises manually activatable inflation devices 6, such as manual inflation means, and means 7 for detecting the pressure in the cushion, such as a pressure gauge.

The support unit comprises at least one pocket 5 configured to house the inflatable cushion 4. Preferably, the pressure of the inflatable cushion 4 is between 80 and 100 millimetres of mercury and the support unit 2 must bear said pressure without deforming in order to transmit said pressure to the patient's shoulder.

The support unit 2 in the form of a jacket comprises a plurality of portions 13 which are stably mutually engageable by means of connections, for example with Velcro; the portions are arranged so as to firmly grip at least one inflatable cushion 4 arranged in the pocket 5 over a shoulder.

The jacket-shaped support unit 2 comprises anchoring means 9, for example strips, adapted to support and hold a patient's arm substantially immobile.

Advantageously, the support unit 2 comprises, at the pocket 5 and in the inner part of the jacket, another pocket 10 for the containment of a material 11, for example memory foam, adapted to uniformly distribute the pressure of the inflatable cushion 4 on the shoulder 3; the pressure of the inflated cushion 4 is thus uniformly distributed over the part where the hematoma is present so as to ensure certain compression values on the entire part of the patient's body affected by the hematoma.

The support unit 2 is applied to the patient so that the same unit adheres to the patient's body, in particular in the area where the inflatable cushion 4 is placed. The latter is initially deflated and is inflated immediately after the application of the support unit 2 to the patient.

Generally, the pressure to be reached is between 80 and 100 millimetres of mercury so as to apply local compression to the patient. The material 11 allows to uniformly distribute said compression exerted by the now inflated cushion 4; moreover, the presence of the material 11 allows the patient to obtain greater comfort.

The pressure in the cushion 4 may be monitored and controlled by the pressure gauge.

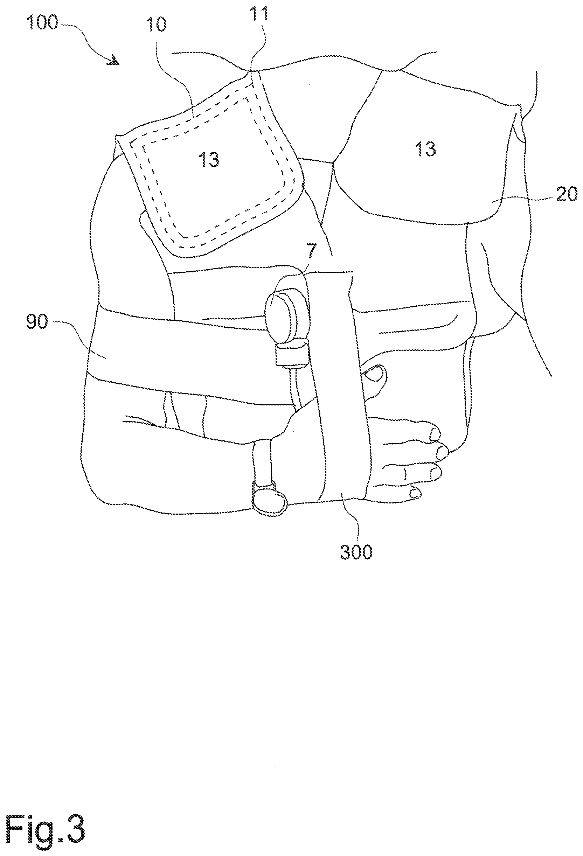

With reference to figures 3-7, a medical apparatus 100 for the containment of hematomas according to a second embodiment of the present invention is described. The medical apparatus 100 of the second embodiment of the invention differs from the medical apparatus 1 of the first embodiment of the invention in the different shape of the inflatable cushion 40 with respect to the inflatable cushion 4 and in the presence of some additional elements 90 and 300.

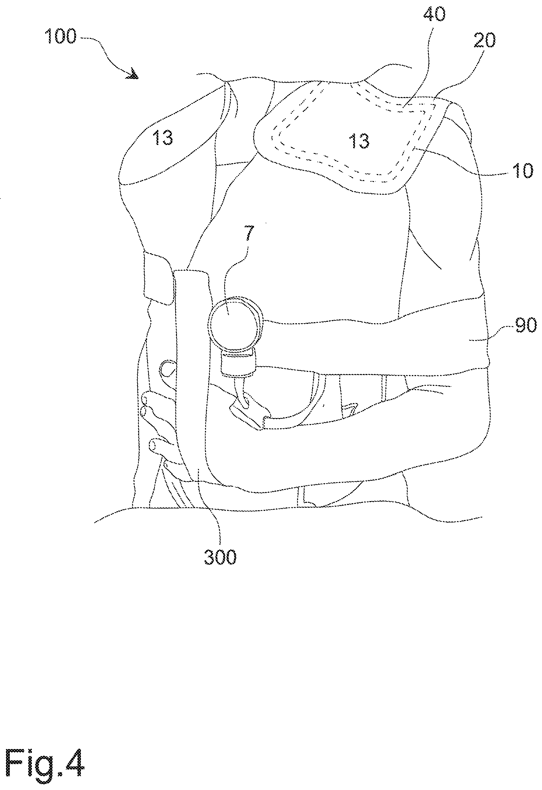

The apparatus 100 comprises a support unit 20, seen in figures 3 and 4, adapted to externally cover and envelop the patient's shoulder; preferably the support unit 20 is in the form of a jacket. The support unit 20 is provided in particular for treatments following operations for implanting a pacemaker or a defibrillator, i.e., when hematomas are possible at the shoulder.

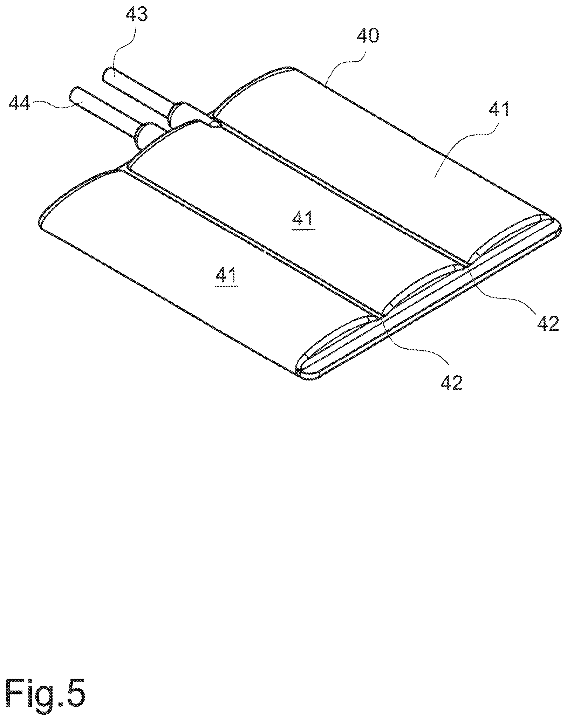

The apparatus comprises at least one bag or cushion 40 preferably inflatable with air, substantially flattened and arranged at or straddling the shoulder, as better seen in figure 5; the inflatable bag or cushion is held in place on the shoulder by the support unit 20. The inflatable cushion 40 is rectangular in shape and has size such as to cover the entire wound caused by the implantation of the pacemaker or defibrillator; the inflatable cushion 40 is designed so as to take a wavy shape when inflated.

In particular, the inflatable cushion 40 comprises a sequence of three sectors 41 having the same dimensions which are normally flattened but when inflated take the top hat-like or -type shape each. The sectors 41 are adjacent to one another and are joined together by long side thereof, i.e., by the cylindrical side surface when the sectors 41 are inflated, by means of junction areas 42 having the same length as the sectors 41; the inflatable cushion 40 has three sectors 41 alternated with two junction areas 42 so as to take the wavy shape once inflated. The sectors 41 are simultaneously inflatable and deflatable by a tube 43 connected to the inflation device 6 while another tube 44 is connected to the pressure detection means 7.

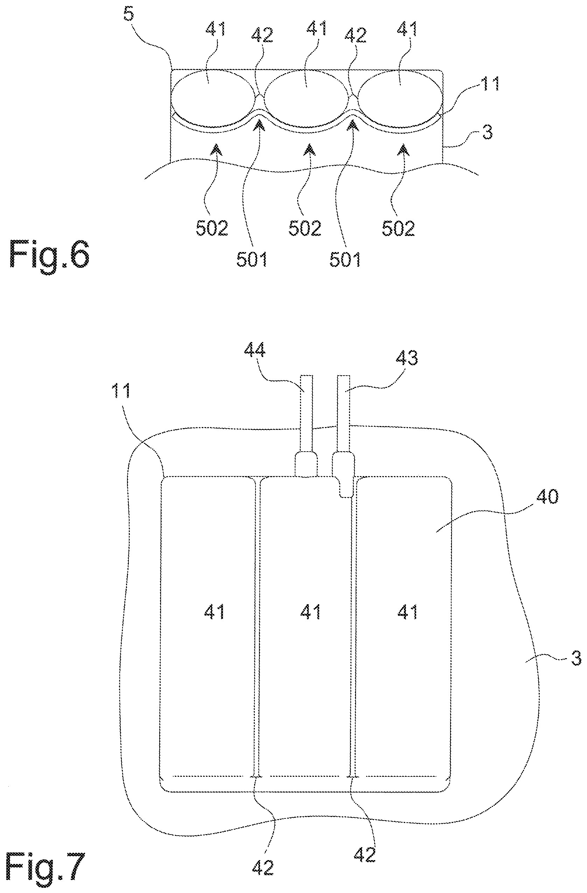

The particular shape of the cushion 40, once inflated, allows the pressure to be well distributed over the entire wound or hematoma of the shoulder 3, however the presence of the junction areas 42 between the sectors 41 allows the formation of areas 501 on the patient's shoulder, i.e., the areas at the junction areas 42, where the pressure exerted by the inflatable cushion is slightly lower than the areas 502 below the sectors 41, as better seen in figures 6 and 7; the cushion 40 thus allows a better circulation of the blood in the areas 501 of the patient's shoulder corresponding to the junction areas 42 as compared to the areas 502 of the patient's shoulder, corresponding to the sectors 41, subjected to greater pressure. Preferably, the sectors 41 are 5 cm wide and 15.7 cm long while the junction areas are 0.25 cm wide and 15.7 cm long.

The apparatus comprises manually activatable inflation devices 6 (not seen in figures 3 and 4), such as manual inflation means, and means 7 for detecting the pressure in the cushion, such as a pressure gauge.

The support unit comprises at least one pocket 5 configured to house the inflatable cushion 40. Preferably, the pressure of the inflatable cushion 40 is between 100 and 150 millimetres of mercury and the support unit 20 must bear said pressure without deforming in order to transmit said pressure to the patient's shoulder.

The support unit 20 in the form of a jacket comprises a plurality of portions 13 which are stably mutually engageable by means of connections, for example with Velcro; the portions are arranged so as to firmly grip at least one inflatable cushion 40 arranged in the pocket 5 over a shoulder. The Velcro engageable portions 13 allow a continuous monitoring of the wound of the patient who has undergone an operation for implanting a pacemaker or defibrillator.

The jacket-shaped support unit 20 comprises means adapted to support and hold a patient's arm substantially immobile. In particular, said means comprise a lateral band 90 with Velcro fastening, which band serves for the immobilization of the limb, thus reducing the complications related to the dislocation of the leads from the cardiac muscle, considering that a pacemaker or defibrillator rests on the pectoral muscle, and any unusual movement of the limb would result in the complication described above, thus making the operation of the implanted devices null.

Advantageously, the support unit 2 comprises, at the pocket 5 and in the inner part of the jacket, another pocket 10 for the containment of the material 11, for example memory foam, for a better patient comfort or ease when the cushion 40 is inflated and exerts compression on the shoulder 3.

The support unit 20 is applied to the patient so that the same unit adheres to the patient's body, in particular in the area where the inflatable cushion 40 is placed. The latter is initially deflated and is inflated immediately after the application of the support unit 20 to the patient.

Generally, the pressure to be reached is between 100 and 150 millimetres of mercury so as to apply local compression to the patient. The material 11 allows the patient to obtain greater comfort.

The pressure in the cushion 40 may be monitored and controlled by the pressure gauge 7. The support unit 20 also comprises a central support 300 for hand housing. This support has been added to prevent and counteract stasis edemas of the affected limb and hand due to the medical contraindication of limb mobility and the operation itself. This support allows to put the limb and hand in relief, preventing edema.

Furthermore, the medical apparatus 100 and components thereof and therefore the inflatable cushion 40 as well are made of radiolucent materials which allow clinical and instrumental investigations on the patient without the need to remove the apparatus itself from the patient; for example, the jacket can be made of cotton.

Figures 3 and 4 show that the apparatus 100 is made to adapt to whether the patient undergoes an implantation on the left or right shoulder, since the pocket 5 for the containment of the inflatable cushion 40 and the pocket 10 for the containment of the material 11 can be placed on the patient's right or left shoulder and always held in place by the support unit 20. Likewise, the band 90 can allow the immobilization of the left or right limb.

According to a variant of the first embodiment of the invention, the cushion 4 can have the same shape as the cushion 40 and be completely the same as the cushion 40; in this case, it does not perform uniform compression on the patient's shoulder where the wound or hematoma is located, but behaves as previously mentioned and shown in figures 6 and 7.

## Claims

1. Medical apparatus (1, 100) for the containment of hematomas for treatment following operations for implanting cardiac devices, said apparatus comprising a support unit (2) in the shape of a jacket, externally enveloping at least a part of the patient's body and adapted to resist efforts aimed to detach said unit from said patient, at least one inflatable cushion (4, 40) held in place over or close to the patient's shoulder by said support unit, **characterized in that** it comprises a material (11) arranged between said inflatable cushion and the patient's shoulder, said material being configured to distribute the pressure of said cushion, once inflated, on the patient's shoulder, said support unit comprising at least one pocket (10) arranged to contain said material (11), said inflatable cushion (40) comprising a sequence of three sectors (41) adjacent to one another and joined together by long side thereof, the inflatable cushion (40) having three sectors (41) alternated with two junction areas (42), so that, once inflated it takes a wavy shape, said three sectors being simultaneously inflatable and deflectable by a tube (43) connected to an inflation device (6) while another tube (44) is connected to a pressure detection means (7), wherein said sectors (41) are 5 cm wide and 15.7 cm long and said junction area (42) are 0.25 cm wide and 15.7 cm long.

2. Medical apparatus according to claim 1, **characterized in that** said material is memory foam.

3. Medical apparatus according to claim 1, **characterized in that** said inflatable cushion is arranged inside a pocket (5) of said support unit.

4. Medical apparatus according to claim 1, **characterized in that** said medical apparatus is made of radiolucent materials.

5. Medical apparatus according to claim 1, **characterized in that** it comprises at least one lateral band (90) for the immobilization of the limb close to said shoulder of the patient.

6. Medical apparatus according to claim 5, **characterized in that** it comprises a central support (300) for the hand of the patient's limb.

7. Medical apparatus according to claim 1, **characterized in that** said support unit (20) comprises a plurality of portions (13) which are stably mutually engageable by means of Velcro connections.

## Patentansprüche

1. Medizinisches Gerät (1, 100) zum Beschränken von Hämatomen zur Behandlung nach Operationen zur Implantation von Herzvorrichtungen, das Gerät umfassend eine Stützeinheit (2), die als eine Jacke ausgebildet ist, die mindestens einen Teil des Körpers des Patienten außen umschließt und eingerichtet ist, Beanspruchungen zu widerstehen, die das Ziel haben, die Einheit von dem Patienten zu lösen, mindestens ein aufblasbares Kissen (4, 40), das durch die Stützeinheit über oder in der Nähe der Schulter des Patienten in Position gehalten wird, **dadurch gekennzeichnet, dass** es ein Material (11) umfasst, das zwischen dem aufblasbaren Kissen und der Schulter des Patienten angeordnet ist, wobei das Material dazu konfiguriert ist, den Druck des Kissens, sobald es aufgeblasen wird, auf der Schulter des Patienten zu verteilen, die Stützeinheit umfassend mindestens eine Tasche (10), die zum Aufnehmen des Materials (11) eingerichtet ist, wobei das aufblasbare Kissen (40) eine Sequenz von drei Abschnitten (41) umfasst, die aneinander angrenzen und durch ihre lange Seite miteinander verbunden sind, wobei das aufblasbare Kissen (40) drei Abschnitte (41) aufweist, die mit zwei Verbindungsbereichen (42) abwechseln, sodass es eine wellenförmige Gestalt annimmt, sobald es aufgeblasen wird, wobei die drei Abschnitte gleichzeitig durch ein Rohr (43), das mit einer Aufblasvorrichtung (6) verbunden ist, aufblasbar und ablassbar sind, während ein anderes Rohr (44) mit einem Druckerkennungsmittel (7) verbunden ist, wobei die Abschnitte (41) 5 cm breit und 15,7 cm lang sind und die Verbindungsbereiche (42) 0,25 cm breit und 15,7 cm lang sind.

2. **.** Medizinisches Gerät nach Anspruch 1, **dadurch gekennzeichnet, dass** das Material Memory-Schaum ist.

3. Medizinisches Gerät nach Anspruch 1, **dadurch gekennzeichnet, dass** das aufblasbare Kissen innerhalb einer Tasche (5) der Stützeinheit angeordnet ist.

4. **.** Medizinisches Gerät nach Anspruch 1, **dadurch gekennzeichnet, dass** das medizinische Gerät aus röntgentransparenten Materialien besteht.

5. **.** Medizinisches Gerät nach Anspruch 1, **dadurch gekennzeichnet, dass** es mindestens ein seitliches Band (90) zur Immobilisierung der Gliedmaße, die sich in der Nähe der Schulter des Patienten befindet.

6. **.** Medizinisches Gerät nach Anspruch 5, **dadurch gekennzeichnet, dass** es eine Zentralstütze (300) für die Hand der Gliedmaße des Patienten umfasst.

7. **.** Medizinisches Gerät nach Anspruch 1, **dadurch gekennzeichnet, dass** die Stützeinheit (20) eine Vielzahl von Abschnitten (13) umfasst, die durch Klettverbindungen stabil miteinander in Eingriff stehen können.

## Revendications

1. Dispositif médical (1, 100) pour le confinement des hématomes destiné au traitement après des opérations d'implantation de dispositifs cardiaques, ledit dispositif comprenant une unité de support (2) en forme de veste, enveloppant extérieurement au moins une partie du corps du patient et adaptée à résister aux efforts visant à détacher ladite unité dudit patient, au moins un coussin gonflable (4, 40) maintenu en place sur ou à proximité de l'épaule du patient par ladite unité de support, **caractérisé en ce qu'**il comprend un matériau (11) disposé entre ledit coussin gonflable et l'épaule du patient, ledit matériau étant configuré pour répartir la pression dudit coussin, une fois gonflé, sur l'épaule du patient, ladite unité de support comprenant au moins une poche (10) agencée pour contenir ledit matériau (11), ledit coussin gonflable (40) comprenant une séquence de trois secteurs (41) adjacents les uns aux autres et reliés ensemble par leur côté longitudinal, le coussin gonflable (40) présentant trois secteurs (41) alternés avec deux zones de jonction (42), de sorte que, une fois gonflé, il prend une forme ondulée, lesdits trois secteurs étant gonflables et déformables simultanément par un tube (43) connecté à un dispositif de gonflage (6) tandis qu'un autre tube (44) est connecté à un moyen de détection de la pression (7), dans lequel lesdits secteurs (41) ont une largeur de 5 cm et une longueur de 15,7 cm et lesdites zones de jonction (42) ont une largeur de 0,25 cm et une longueur de 15,7 cm.

2. **.** Dispositif médical selon la revendication 1, **caractérisé en ce que** ledit matériau est une mousse à mémoire de forme.

3. Dispositif médical selon la revendication 1, **caractérisé en ce que** ledit coussin gonflable est disposé à l'intérieur d'une poche (5) de ladite unité de support.

4. **.** Dispositif médical selon la revendication 1, **caractérisé en ce que** ledit dispositif médical est réalisé en matériaux radiotransparents.

5. **.** Dispositif médical selon la revendication 1, **caractérisé en ce qu'**il comprend au moins une bande latérale (90) pour l'immobilisation du membre proche de ladite épaule du patient.

6. **.** Dispositif médical selon la revendication 5,
**caractérisé en ce qu'**il comprend un support central (300) pour la main du membre du patient.

7. **.** Dispositif médical selon la revendication 1, **caractérisé en ce que** ladite unité de support (20) comprend une pluralité de portions (13) pouvant être fixées de manière stable entre elles au moyen de connexions en Velcro.
